(19) Europäisches Patentamt
European Patent Office
Office européen des brevets

(11) Veröffentlichungsnummer: **0 372 329**
**A2**

# (12) EUROPÄISCHE PATENTANMELDUNG

(21) Anmeldenummer: **89121782.0**

(51) Int. Cl.[5]: **C07D 401/12, A01N 43/54**

(22) Anmeldetag: **24.11.89**

Patentansprüche für folgenden Vertragsstaat: ES.

(30) Priorität: **09.12.88 DE 3841432**

(43) Veröffentlichungstag der Anmeldung:
**13.06.90 Patentblatt 90/24**

(84) Benannte Vertragsstaaten:
**AT BE CH DE ES FR GB IT LI NL SE**

(71) Anmelder: **BASF Aktiengesellschaft**
**Carl-Bosch-Strasse 38**
**D-6700 Ludwigshafen(DE)**

(72) Erfinder: **Rheinheimer, Joachim, Dr.**
**Mehrziger Strasse 24**
**D-6700 Ludwigshafen(DE)**
Erfinder: **Eicken, Karl, Dr.**
**Am Huetteningert 12**
**D-6706 Wachenheim(DE)**
Erfinder: **Plath, Peter, Dr.**
**Hans-Balcke-Strasse 13**
**D-6710 Frankenthal(DE)**
Erfinder: **Westphalen, Karl-Otto, Dr.**
**Mausbergweg 58**
**D-6720 Speyer(DE)**
Erfinder: **Wuerzer, Bruno, Dr.**
**Ruedigerstrasse 13**
**D-6701 Otterstadt(DE)**

## (54) Pyridinderivate und ihre Verwendung als herbizide Wirkstoffe.

(57) Die Erfindung betrifft neue Pyridinderivate, Verfahren zu ihrer Herstellung und ihre Verwendung zur Bekämpfung unerwünschten Pflanzenwuchses.

Die Pyridinderivate haben die Formel

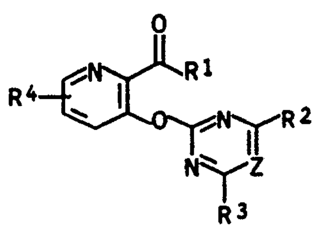

in der
$R^1$ für unsubstituiertes oder durch Alkoxi, Alkylthio oder Phenyl ein- oder zweifach substituiertes Alkylidenaminoxi, unsubstituiertes oder durch ALkyl ein- oder zweifach substituiertes Cycloalkylidenaminoxi, Succinyliminoxi, unsubstituiertes oder durch Alkyl oder Halogen ein- oder zweifach substituiertes Azolyl,
$R^2$, $R^3$ für Alkyl, Halogenalkyl, Alkoxi, Halogenalkoxi oder Alkylthio,
$R^4$ für Wasserstoff, Halogen, Alkyl, Halogenalkyl oder Alkoxi und
Z für Stickstoff oder die Methingruppe stehen.

EP 0 372 329 A2

## Pyridinderivate und ihre Verwendung als herbizide Wirkstoffe

Die vorliegende Erfindung betrifft neue Pyridinderivate, Verfahren zu ihrer Herstellung und ihre Verwendung zur Bekämpfung unerwünschten Pflanzenwuchses.

Herbizide Picolinsäurederivate sind aus der EP-A-249 707 bekannt.

Es wurden nun neue Pyridinderivate der Formel I

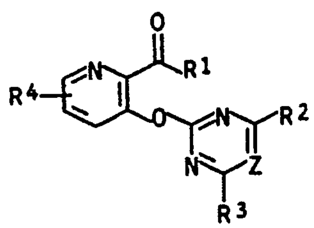

(I)

gefunden, in der

$R^1$ für unsubstituiertes oder durch Alkoxi, Alkylthio oder Phenyl ein- oder zweifach substituiertes Alkylidenaminoxi, unsubstituiertes oder durch Alkyl ein- oder zweifach substituiertes Cycloalkylidenaminoxi, Succinyliminoxi, unsubstituiertes oder durch Alkyl oder Halogen ein- oder zweifach substituiertes Azolyl,

$R^2$, $R^3$ für Alkyl, Halogenalkyl, Alkoxi, Halogenalkoxi oder Alkylthio,

$R^4$ für Wasserstoff, Halogen, Alkyl, Halogenalkyl oder Alkoxi und

Z für Stickstoff oder die Methingruppe stehen.

Diese Verbindungen sind herbizid wirksam.

In Formel I haben die Substituenten $R^1$, $R^2$, $R^3$, $R^4$ und Z die folgenden Bedeutungen:

$R^1$ bedeutet beispielsweise symmetrisches oder unsymmetrisches, unsubstituiertes oder durch $C_1$-$C_3$-Alkylthio, $C_1$-$C_3$-Alkoxi oder Phenyl substituiertes $C_3$-$C_{20}$-, vorzugsweise $C_3$-$C_{15}$-Alkylidenaminoxi, gegebenenfalls durch Methyl substituiertes $C_4$-$C_{12}$-, vorzugsweise $C_5$-$C_8$-Cycloalkylidenaminoxi, Succinyliminoxi oder unsubstituiertes oder durch Chlor oder Methyl substituiertes Azolyl.

Die Substituenten $R^2$ und $R^3$ können gleich oder verschieden sein und bedeuten $C_1$-$C_5$-Alkyl, $C_1$-$C_5$-Halogenalkyl, z.B. $C_1$-$C_3$-Chlor- oder Fluoralkyl, $C_1$-$C_5$-Alkoxi, $C_1$-$C_5$-Halogenalkoxi, z.B. $C_1$-$C_2$-Chlor- oder Fluoralkoxi oder $C_1$-$C_4$-Alkylthio.

$R^4$ steht für Halogen, wie Chlor, Fluor, Brom, $C_1$-$C_6$-Alkyl, $C_1$-$C_6$-Halogenalkyl, z.B. $C_1$-$C_2$-Chlor- oder Fluoralkyl, oder für $C_1$-$C_5$-Alkoxi.

Unter Alkyl oder Alkyl in einer Alkoxigruppe oder einer Alkylthiogruppe ist je nach der genannten Zahl der Kohlenstoffatome beispielsweise Methyl, Ethyl, Propyl, Isopropyl, Butyl, sek.-Butyl, tert.-Butyl, Pentyl und Isomere, Hexyl und Isomere, Heptyl und Isomere oder Octyl und Isomere zu verstehen. Entsprechend sind auch bei den höheren Homologen die Isomeren jeweils mit einbezogen. Cycloalkyliden bedeutet beispielsweise Cyclobutyliden, Cyclopentyliden, Cyclohexyliden, Cycloheptyliden oder Cyclooctyliden. Azolyl kann Imidazolyl, Pyrazolyl oder Triazolyl bedeuten.

Von den Verbindungen der Formel I sind solche Gruppen besonders zu erwähnen, bei denen

$R^1$ für $C_5$-$C_8$-Cycloalkylidenaminoxi, unsubstituiertes oder durch Methoxi, Phenyl oder Methylthio substituiertes $C_2$-$C_{10}$-Alkylidenaminoxi, Imidazolyl, Pyrazolyl oder Triazolyl,

$R^2$, $R^3$ für $C_1$-$C_3$-Alkyl, Trifluormethyl, $C_1$-$C_3$-Alkoxi, Difluormethoxi oder $C_1$-$C_3$-Alkylthio, insbesondere für $C_1$-$C_3$-Alkoxi,

$R^4$ für Wasserstoff, Fluor, Chlor, Brom, $C_1$-$C_4$-Alkyl oder $C_1$-$C_4$-Alkoxi, insbesondere für Wasserstoff,

stehen. Besonders wirksam sind Verbindungen, bei denen Z die Methingruppe ist.

Die Pyridinderivate der Formel I lassen sich durch Umsetzung eines Picolinsäurederivats der Formel II

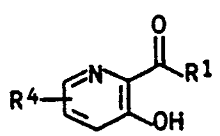

(II)

in der $R^1$ Hydroxy bedeutet und $R^4$ die für Formel I genannten Bedeutungen hat, mit einem Heterocyclus

der Formel III

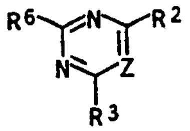

(III)

in an sich bekannter Weise erhalten.

Anschließend überführt man die so erhaltene heterocyclisch substituierte Carbonsäure nach bekannten Methoden über eine aktivierte Zwischenstufe -wie z.B. ein Halogenid oder ein gemischtes Anhydrid - in die Pyridinderivate der Formel I. Diese Umsetzung läßt sich auch ohne eine Aktivierung der Carbonsäure in Gegenwart eines wasserabspaltenden Mittels, wie z.B. eines Carbodiimids, durchführen.

In Formel III bedeutet $R^5$ Chlor, Brom, Iod, Arylsulfonyl, Alkylsulfonyl, wie z.B. Toluolsulfonyl oder Methylsulfonyl, oder eine andere äquivalente Abgangsgruppe.

Ein anderer Weg zur Herstellung der Pyridinderivate der Formel I besteht in der Umsetzung eines Picolinsäurederivates der Formel II, in der $R^1$ und $R^4$ die für Formel I genannten Bedeutungen haben, mit einem Heterocyclus der Formel III in Gegenwart einer Base.

Die Verbindungen der Formel III mit einem reaktionsfähigen Substituenten $R^5$ sind größtenteils bekannt oder aber in üblicher Weise ohne Schwierigkeiten herstellbar. Das gleiche gilt für die Picolinsäurederivate der Formel II.

Als Base können Alkali- oder Erdalkalimetallhydride, wie NaH oder $CaH_2$, Alkalimetallhydroxide, wie NaOH oder KOH, Alkalimetallalkoholate, wie Kalium-tert.-butylat, Alkalimetallcarbonate, wie $Na_2CO_3$ oder $K_2CO_3$, Alkalimetallamide, wie $NaNH_2$ oder Lithiumdiisopropylamid, oder tertiäre Amine Verwendung finden. Bei Einsatz einer anorganischen Base kann man einen Phasentransferkatalysator zusetzen, wenn dies den Umsatz fördert. Die Menge an Base beträgt zweckmäßigerweise 1 bis 20 Mol, bezogen auf ein Mol Picolinsäurederivat II, vorzugsweise, je nach verwendeter Base, 1 bis 3 Mol.

Herstellungsbeispiel

Allgemeine Vorschrift zur Herstellung aromatischer Carbonsäureoximester der Formel I:

6,4 mmol des Kaliumsalzes der jeweiligen 3-(4,6-Dimethoxypyrimidin-2-yl)oxipicolinsäure werden in 40 ml Dimethoxyethan vorgelegt, auf 0 °C gekühlt und mit 7,0 mmol Oxalylchlorid versetzt. Man rührt bei 0 °C 1 h nach und verdampft dann etwa 30 % des Lösungsmittels im Vakuum. Man gibt nun 7,6 mmol des jeweiligen Oxims und 6,4 mmol Pyridin zu und rührt über Nacht bei Raumtemperatur. Man gießt in verdünnte Essigsäure, extrahiert mit Essigsäureethylester und wäscht mit verdünnter Essigsäure. Die organische Phase wird über Natriumsulfat getrocknet und im Vakuum eingeengt. Die zurückbleibende Substanz kann beispielsweise durch Chromatographie an Silica-Gel oder durch Umkristallisation aus einem üblichen Lösungsmittel weiter gereinigt werden.

Nach dieser Vorschrift können die Verbindungen der Formel I durch Wahl entsprechender Ausgangsstoffe hergestellt werden. Beispiele für Pyridinderivate der Formel I sind in Tabelle 1 aufgeführt.

Tabelle 1

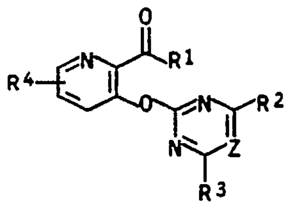

(I) .

| | $R^1$ | $R^2$ | $R^3$ | $R^4$ | Z | [°C]/ δ: $^1$H-NMR [ppm] |
|---|---|---|---|---|---|---|
| 1 | 2-Propaniminoxi | $OCH_3$ | $OCH_3$ | H | CH | 124-125 |
| 2 | Cyclohexaniminoxi | $OCH_3$ | $OCH_3$ | H | CH | 104-109 |
| 3 | 2-Methylcylcohexaniminoxi | $OCH_3$ | $OCH_3$ | H | CH | |
| 4 | 2-Propaniminoxi | $OCH_3$ | $OCH_3$ | H | N | |
| 5 | 2-Propaniminoxi | $SCH_3$ | $SCH_3$ | H | N | |
| 6 | 1-Methoxipropan-2-iminoxi | $OCH_3$ | $OCH_3$ | H | CH | 76-78 |
| 7 | 3-Methylthiobutan-2-iminoxi | $OCH_3$ | $OCH_3$ | H | CH | 91-94 |
| 8 | 1-Phenylethan-1-iminoxi | $OCH_3$ | $OCH_3$ | H | CH | 112-114 |
| 9 | 2-Dodecaniminoxi | $OCH_3$ | $OCH_3$ | H | CH | |
| 10 | 2-Methylhexan-3-iminoxi | $OCH_3$ | $OCH_3$ | H | CH | 98-99 |
| 11 | 1-Imidazolyl | $OCH_3$ | $OCH_3$ | H | CH | |
| 12 | 1-([1,2,4]-Triazolyl) | $OCH_3$ | $OCH_3$ | H | CH | |
| 13 | 1-Pyrazolyl | $OCH_3$ | $OCH_3$ | H | CH | |
| 14 | 2-Propaniminoxi | $CH_3$ | $OCH_3$ | H | CH | |
| 15 | 2-Propaniminoxi | $CF_3$ | $OCH_3$ | H | CH | |
| 16 | 2-Propaniminoxi | $OCHF_2$ | $OCHF_2$ | H | CH | |
| 17 | 2-Propaniminoxi | $OCH_3$ | $OCH_3$ | 6-$CH_3$ | CH | |
| 18 | 3-Dodecaniminoxi | $OCH_3$ | $OCH_3$ | H | CH | 0,80-1,60 (m; 20H); 2,26-2,50 (m; 4H); 3,80 (s; 6H); 5,77 (s; 1H) |

Die Pyridinderivate der Formel I bzw. die sie enthaltenden Mittel sind herbizid wirksam.

Die Verbindungen der Formel I bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen aus Mineralölfraktionen von mittlerem bis hohem Siedepunkt, wie Kerosin oder Dieselöl, ferner aus Kohlenteerölen sowie aus Ölen pflanzlichen oder tierischen Ursprungs, aus

aliphatischen, cyclischen und aromatischen Kohlenwasserstoffen, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron, oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali- und Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl, Octyl- oder Nonylphenol, Alkylphenol-, Tri butylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Beispiele für Formulierungen sind:

I. Man vermischt 90 Gewichtsteile der Verbindung Nr. 5 mit 10 Gewichtsteilen N-methyl-α-pyrrolidon und erhält eine Lösung, die zur Anwendung in Form kleinster Tropfen geeignet ist.

II. 20 Gewichtsteile der Verbindung Nr. 1 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 4 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 14 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 16 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 1 werden mit einer Mischung aus 92 Gewichtsteilen

pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 5 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadien 0,001 bis 3 vorzugsweise 0,01 bis 1 kg/ha.

In Anbetracht der Vielseitigkeit der Applikationsmethoden können die erfindungsgemäßen Verbindungen bzw. sie enthaltende Mittel in einer Reihe von Kulturpflanzen zur Beseitigung unerwünschter Pflanzen eingesetzt werden.

Zur Verbreiterung des Wirkungsspektrums und zur Erzielung synergistischer Effekte können die Pyridinderivate der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, (Hetero)-Aryloxyphenoxypropionsäure(derivate) und andere in Betracht.

Außerdem kann es von Nutzen sein, die Pyridinderivate der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopatogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Beispiele zur biologischen Wirkung

Der Einfluß verschiedener Vertreter der erfindungsgemäßen Pyridinderivate der Formel I auf das Wachstum von erwünschten und unerwünschten Pflanzen wird durch die folgenden biologischen Beispiele aus Gewächshausversuchen belegt:

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt eingesät.

Zum Zweck der Nachauflaufbehandlung werden entweder direkt gesäte oder in den gleichen Gefäßen aufgewachsene Pflanzen ausgewählt oder sie werden erst als Keimpflanze getrennt angezogen und eineige Tage vor der Behandlung in die Versuchsgefäße verpflanzt.

Die in den Gewächshausversuchen verwendeten Pflanzen setzen sich aus folgenden Arten zusammen:

| Lateinischer Name | Deutscher Name |
|---|---|
| Abutilon theophrasti | Chinesischer Hanf |
| Amaranthus retroflexus | Zurückgekrümter Fuchsschwanz |
| Chenopodium album | Weißer Gänsefuß |
| Malva neglecta | Wegmalve |
| Solanum nigrum | Schwarzer Nachtschatten |
| Stellaria media | Vogelsternmiere |

Je nach Wuchsform werden die Testpflanzen bei einer Wuchshöhe von 3 - 15 cm dann mit den in Wasser als Verteilungsmittel suspendierten oder emulgierten Wirkstoffen, die durch fein verteilende Düsen gespritzt werden, behandelt. Die Aufwandmenge für die Nachauflaufbehandlung beträgt 0,06 kg/ha a.S.

Die Versuchsgefäße werden im Gewächshaus aufgestellt, wobei für wärmeliebende Arten wärmere Bereiche (20 bis 35° C) und für solche gemäßigter Klimate 10 bis 20° C bevorzugt werden. Die Versuchsperiode erstreckt sich über 2 bis 4 Wochen. Während dieser Zeit werden die Pflanzen gepflegt und ihre Reaktion auf die einzelnen Behandlungen wird ausgewertet.

Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Bei Nachauflaufanwendung lassen sich beispielsweise mit den Wirkstoffen Nr. 1 und Nr. 2 breitblättrige unerwünschte Pflanzen sehr gut bekämpfen.

## Ansprüche

1. Pyridinderivate der Formel I

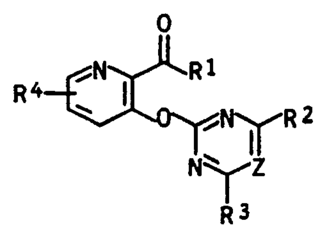

(I),

in der

$R^1$ für unsubstituiertes oder durch Alkoxi, Alkylthio oder Phenyl ein- oder zweifach substituiertes Alkylidenaminoxi, unsubstituiertes oder durch ALkyl ein- oder zweifach substituiertes Cycloalkylidenaminoxi, Succinyliminoxi, unsubstituiertes oder durch Alkyl oder Halogen ein- oder zweifach substituiertes Azolyl,

$R^2$, $R^3$ für Alkyl, Halogenalkyl, Alkoxi, Halogenalkoxi oder Alkylthio,

$R^4$ für Wasserstoff, Halogen, Alkyl, Halogenalkyl oder Alkoxi und

Z für Stickstoff oder die Methingruppe stehen.

2. Pyridinderivate der Formel I gemäß Anspruch 1, dadurch gekennzeichnet, daß Z für die Methingruppe steht.

3. Verfahren zur Herstellung von Pyridinderivaten gemäß Anspruch 1, dadurch gekennzeichnet, daß man Pyridin-2-carbonsäurederivate der Formel II

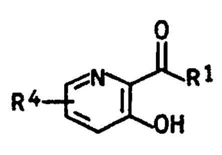

(II)

mit einem Heterocyclus der Formel III

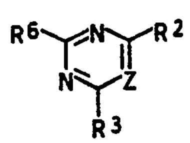

(III)

in der $R^6$ für Halogen, Alkylsulfonyloxi, Arylsulfonyloxi oder eine andere äquivalente Abgangsgruppe steht, in Gegenwart einer anorganischen oder organischen Base umsetzt.

4. Herbizides Mittel, gekennzeichnet durch einen Gehalt an mindestens einem Pyridinderivat der Formel I gemäß Anspruch 1.

5. Herbizides Mittel, enthaltend inerte Zusatzstoffe und ein Pyridinderivat der Formel I gemäß Anspruch 1.

6. Verfahren zur Bekämpfung unerwünschten Pflanzenwuchses, dadurch gekennzeichnet, daß man eine herbizid wirksame Menge eines Pyridinderivats der Formel I gemäß Anspruch 1 auf die Pflanzen oder

deren Lebensraum einwirken läßt.

Patentansprüche für folgenden Vertragsstaat: ES

1. Verfahren zur Herstellung von Pyridinderivaten der Formel I

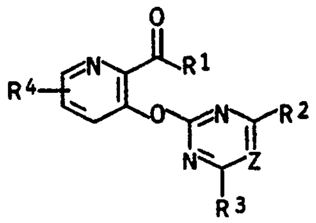

(I),

in der
$R^1$ für unsubstituiertes oder durch Alkoxi, Alkylthio oder Phenyl ein- oder zweifach substituiertes Alkylidenaminoxi, unsubstituiertes oder durch ALkyl ein- oder zweifach substituiertes Cycloalkylidenaminoxi, Succinyliminoxi, unsubstituiertes oder durch Alkyl oder Halogen ein- oder zweifach substituiertes Azolyl,
$R^2$, $R^3$ für Alkyl, Halogenalkyl, Alkoxi, Halogenalkoxi oder Alkylthio,
$R^4$ für Wasserstoff, Halogen, Alkyl, Halogenalkyl oder Alkoxi und
Z für Stickstoff oder die Methingruppe stehen,
dadurch gekennzeichnet, daß man Pyridin-2-carbonsäurederivate der Formel II

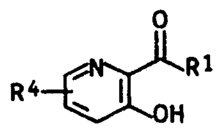

(II)

mit einem Heterocyclus der Formel III

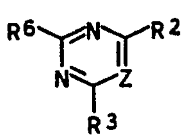

(III)

in der $R^6$ für Halogen, Alkylsulfonyloxi, Arylsulfonyloxi oder eine andere äquivalente Abgangsgruppe steht, in Gegenwart einer anorganischen oder organischen Base umsetzt.

2. Herbizides Mittel, enthaltend inerte Zusatzstoffe und ein Pyridinderivat der Formel I gemäß Anspruch 1.